# EUROPEAN PATENT APPLICATION

(11) **EP 4 567 019 A1**
(43) Date of publication of application: **11.06.2025**
(21) Application number: 23849907.3
(22) Date of filing: 20.07.2023
(51) Int. Cl.: C07C 25/24, C07C 17/263, C08G 77/52

(54) **FLUORENE COMPOUND HAVING HALOGEN ATOM AND ALLYL GROUP, AND METHOD FOR PRODUCING SAME**

(30) Priority: 03.08.2022 JP 2022123960
(71) Applicant: SHIN-ETSU CHEMICAL CO., LTD., Tokyo 1000005 (JP)
(72) Inventor: OMORI, Hiroto, Annaka-shi Gunma 379-0224 (JP); YANAGISAWA, Hideyoshi, Annaka-shi Gunma 379-0224 (JP)
(74) Representative: Ter Meer Steinmeister & Partner
(86) International application number: PCT/JP2023/026543
(87) International publication number: WO 2024/029352

(57) **Abstract**

Provided is a fluorine compound that is represented by formula (1) and that has a halogen atom and an allyl group. 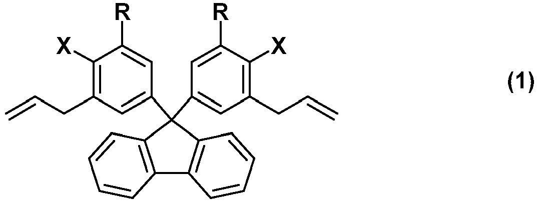 (In the formula, each R independently represents a hydrogen atom or a methyl group. Each X independently represents a fluorine atom, a chlorine atom, a bromine atom, or an iodine atom.)

## Description

### TECHNICAL FIELD

This invention relates to a fluorene compound having halogen atoms and allyl groups and a method for preparing the same.

### BACKGROUND ART

As one of prior art fluorene compounds having allyl groups, Patent Document 1 describes the compound having allyl and epoxy groups, represented by the following formula (X), which is known as a functional epoxy resin. It is ascertained that the epoxy resin having a fluorene skeleton introduced therein is improved in heat resistance and electrical properties over prior art epoxy resins.

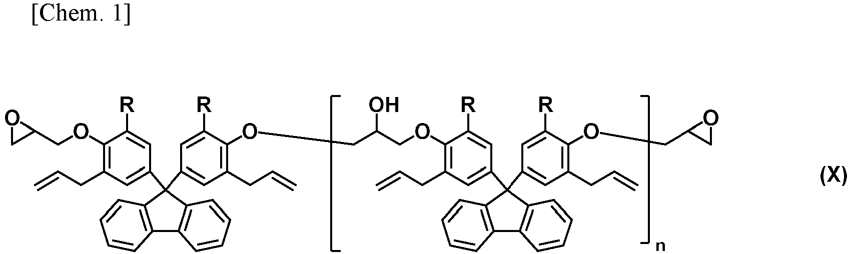

Since the introduction of fluorene skeletons into resins is effective for improving heat resistance and electrical properties, fluorene compounds are important for the development of various functional resins. Inter alia, fluorene compounds having allyl groups are quite important in that polymers can be readily synthesized through such reaction as hydrosilation reaction.

As the fluorene compounds having allyl groups, Patent Documents 1 to 3 describe compounds having epoxy or phenolic hydroxy groups. For the development of various functional resins, compounds having halogen atoms which can be converted to various substituents are desired. Further, fluorene compounds having halogen atoms and allyl groups are expected to shorten the aging time taken for the synthesis of polymers through hydrosilation reaction because hydrosilation reaction is promoted by halogen atoms.

### PRIOR ART DOCUMENTS

### PATENT DOCUMENTS

| | |
|---|---|
| Patent Document 1: | JP 4873223 |
| Patent Document 2: | JP-A 2014-047207 |
| Patent Document 3: | JP-A 2014-062055 |

### SUMMARY OF INVENTION

### TECHNICAL PROBLEM

An object of the invention, which has been made under the above-mentioned circumstances, is to provide a fluorene compound having halogen atoms and allyl groups which can be a precursor to various fluorene skeleton-containing resins, and a method for preparing the same.

### SOLUTION TO PROBLEM

Making extensive investigations to attain the above object, the inventors have found that the outstanding problems can be solved by a fluorene compound having halogen atoms and allyl groups. The invention is predicated on this finding.

The invention provides a fluorene compound having halogen atoms and allyl groups and a method for preparing the same, as defined below.
1. A fluorene compound having halogen atoms and allyl groups, represented by the formula (1): 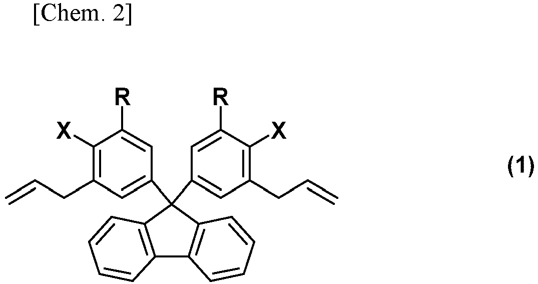 wherein R is each independently hydrogen or methyl and X is each independently fluorine, chlorine, bromine or iodine.
2. The fluorene compound of 1 wherein both R are hydrogen.
3. A method for preparing a fluorene compound represented by the formula (1), comprising the step of reacting fluorenone represented by the formula (2) with a compound represented by the formula (3), 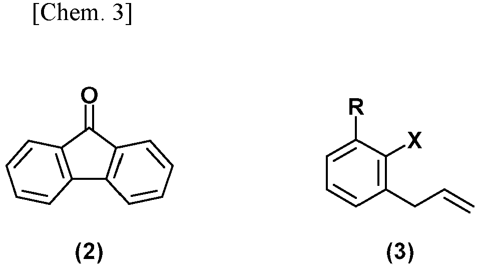 wherein R is hydrogen or methyl and X is fluorine, chlorine, bromine or iodine, 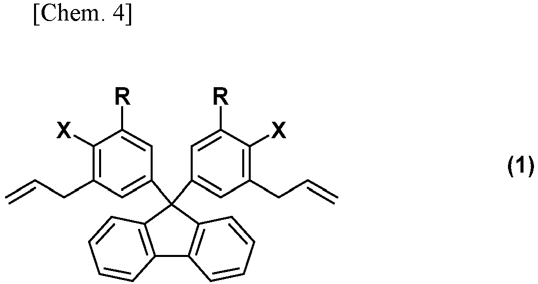 wherein R and X are as defined above.
4. A method for preparing a fluorene skeleton-containing organosilicon compound comprising the step of effecting hydrosilation reaction of the fluorene compound of 1 or 2 with an organosilicon compound containing a Si-H group in the presence of a catalyst.

### ADVANTAGEOUS EFFECTS OF INVENTION

The fluorene compound having halogen atoms and allyl groups according to the invention is a useful precursor for the development of new fluorene skeleton-containing functional resins because it allows for easy introduction of substituent groups which are different from the groups known for prior art fluorene compounds having allyl groups.

### DESCRIPTION OF EMBODIMENTS

One embodiment of the invention is a fluorene compound having halogen atoms and allyl groups, represented by the formula (1), which is simply referred to as "fluorene compound," hereinafter.

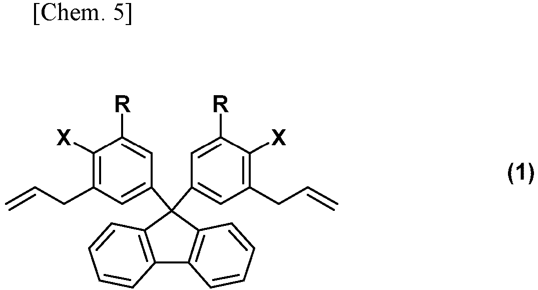

In formula (1), R is each independently hydrogen or methyl, preferably hydrogen for availability of starting reactants.

In formula (1), X is each independently fluorine, chlorine, bromine or iodine, preferably chlorine or bromine.

The fluorene compound can be synthesized by reacting fluorenone represented by the formula (2) with a compound represented by the formula (3). 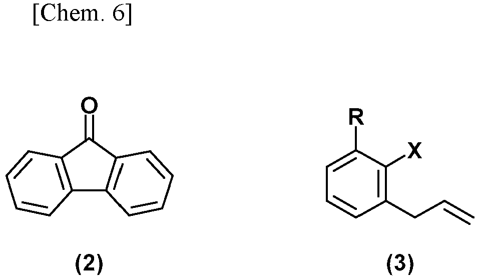 Herein R and X are as defined above.

For the reaction of the compound having formula (2) with the compound having formula (3), any of methods known in the art, for example, the methods described in JP-A 2017-114947, JP-A 2020-075866, and JP 6936544 may be applied. The reaction temperature is typically 0°C to 150°C, though arbitrary, and the reaction time is typically about 2 to about 30 hours.

Although the amounts of the compound having formula (2) and the compound having formula (3) used are arbitrary, the amount of the compound having formula (3) used is typically 2.0 to 30 moles per mole of the compound having formula (2). The amount of the compound having formula (3) is preferably 2.0 to 20 moles, more preferably 2.0 to 10 moles per mole of the compound having formula (2) because the compound having formula (1) is obtained in high yields.

In the reaction, an acid may be added. Examples of the acid include hydrochloric acid, sulfuric acid, nitric acid, phosphoric acid, acetic acid, trifluoroacetic acid, and p-toluenesulfonic acid, but are not limited thereto. The amount of the acid used is preferably 0.01 to 10 moles, more preferably 0.05 to 3 moles per mole of the compound having formula (2). The acid may be used alone or in admixture of two or more.

In the reaction, a solvent may be used. The solvent is preferably selected from carbon tetrachloride, carbon disulfide, dichloromethane, chloroform, and nitromethane. The amount of the solvent used is typically 30 to 500 parts by weight, preferably 50 to 300 parts by weight per 100 parts by weight of the compound having formula (2).

The reaction product resulting from the above reaction is washed with water and heated under reduced pressure to remove the solvent or the like, obtaining the desired fluorene compound. In the washing step, any of aqueous solutions of metal hydroxides such as sodium hydroxide and potassium hydroxide and metal carbonates or hydrogencarbonates such as sodium carbonate, sodium hydrogencarbonate and potassium carbonate may be used.

For utilization of allyl groups, the fluorene compound can be subjected to hydrosilation reaction with an organosilicon compound containing a Si-H group. Although the amounts of the fluorene compound and the SiH-containing organosilicon compound used in the hydrosilation reaction are not particularly limited, 0.9 to 0.99 mole of the SiH-containing organosilicon compound is preferably used per mole of the fluorene compound. A polymer having a high molecular weight is then obtained.

Examples of the SiH-containing organosilicon compound include, but are not limited to, hydrosilanes such as trimethoxyhydrosilane, triethoxyhydrosilane and triethylsilane; dimethylpolysiloxanes containing Si-H groups at both ends such as 1,1,3,3-tetramethyldisiloxane, α,ω-dihydrodimethylpolysiloxane and α,ω-dihydromethylphenylpolysiloxane; dimethylpolysiloxanes containing a Si-H group at one end such as 1,1,3,3,3-pentamethyldisiloxane and 1,1,1,3,3,5,5-heptamethyltrisiloxane; dimethylpolysiloxanes containing a Si-H group on side chain such as 1,1,1,3,5,5,5-heptamethyltrisiloxane; methylphenylpolysiloxanes containing a Si-H group on side chain such as 1,1,1,5,5,5-hexamethyl-3-phenyltrisiloxane; cyclic polysiloxanes containing a Si-H group such as 2,4,6-trimethylcyclotrisiloxane and 2,4,6,8-tetramethylcyclotetrasiloxane; branched polysiloxanes containing a Si-H group; methylsilicone resins containing a Si-H group; methylphenylsilicone resins containing a Si-H group; silphenylene compounds containing a Si-H group; and silalkylene compounds containing a Si-H group.

In the hydrosilation reaction of the fluorene compound with the organosilicon compound containing a Si-H group, another vinyl-containing compound may be co-reacted if desired. Examples of the other vinyl-containing compound include dimethylpolysiloxanes containing vinyl groups at both ends such as 1,3-divinyl-1,1,3,3-tetramethyldisiloxane, α,ω-divinyldimethylpolysiloxane and α,ω-divinylmethylphenylpolysiloxane; dimethylpolysiloxanes containing a vinyl group on side chain such as 1,1,1,3,5,5,5-heptamethyl-3-vinyltrisiloxane; methylvinylphenylpolysiloxanes containing a vinyl group on side chain such as 1,1,1,5,5,5-hexamethyl-3-vinyl-3-phenyltrisiloxane; vinyl-containing cyclic polysiloxanes such as 2,4,6-trimethyl-2,4,6-trivinylcyclotrisiloxane and 2,4,6,8-tetramethyl-2,4,6,8-tetravinylcyclotetrasiloxane; vinyl-containing branched polysiloxanes, methylvinylsilicone resins, and methylvinylphenylsilicone resins.

The hydrosilation reaction is carried out in the presence of a catalyst. Examples of the catalyst which can be used herein include platinum group metal elements such as platinum (inclusive of platinum black), rhodium and palladium; platinum chloride, chloroplatinic acid and chloroplatinic acid salts such as H₂PtCl₄·xH₂O, H₂PtCl₆·xH₂O, NaHPtCl₆·xH₂O, KHPtCl₆·xH₂O, Na₂PtCl₆·xH₂O, K₂PtCl₄·xH₂O, PtCl₄·xH₂O, PtCl₂, and Na₂HPtCl₄·xH₂O wherein x is preferably an integer of 0 to 6, more preferably 0 or 6; alcohol-modified chloroplatinic acid as described in USP 3,220,972; complexes of chloroplatinic acid with olefins as described in USP 3,159,601, USP 3,159,662 and USP 3,775,452; platinum group metals such as platinum black and palladium on supports such as alumina, silica and carbon; rhodium-olefin complexes; chlorotris(triphenylphosphine)rhodium, known as Wilkinson catalyst; and complexes of platinum chloride, chloroplatinic acid or chloroplatinic acid salts with vinyl-containing siloxanes (specifically, vinyl-containing cyclic siloxanes).

The catalyst is used in a catalytic amount. In most cases, the amount of the catalyst is preferably 0.001 to 0.1% by weight of platinum group metal based on the total amount of the reaction polymer. In the polymerization reaction, a solvent may be used if necessary. Preferred examples of the solvent include hydrocarbon solvents such as toluene and xylene. The amount of the solvent, though not particularly limited, is preferably 30 to 1,000 parts by weight (i.e., 0.3 to 10 times greater) per 100 parts by weight of the fluorene compound and the SiH-containing organosilicon compound combined. Since the polymerization conditions are normally set such that the catalyst may not be deactivated and polymerization be completed in a short time, the polymerization temperature is, for example, 40 to 150°C, preferably 60 to 120°C. As to the polymerization time which varies with the type and amount of the polymer, the polymerization is preferably completed within 0.5 to 100 hours, especially 0.5 to 30 hours in order to avoid the entry of moisture into the polymerization system. At the end of polymerization reaction, the solvent if any is distilled off, obtaining the silicon-containing fluorene compound.

Since the inventive fluorene compound has halogen atoms which can be readily converted to other substituent groups, it can be a precursor to synthesize fluorene compounds having allyl groups and substituent groups which are different from the groups introduced in prior art fluorene compounds having allyl groups. The inventive fluorene compound is useful for the development of fluorene skeleton-containing resin materials.

Since the fluorene compound having halogen atoms and allyl groups is highly reactive for hydrosilation, the time taken for the synthesis of polymers by hydrosilation can be shortened as compared with the use of halogen-free fluorene compounds having allyl groups.

### EXAMPLES

Examples and Comparative Examples are given below for illustrating the invention although the invention is not limited thereto. In Examples and Comparative Examples, Mw is measured by gel permeation chromatography (GPC) versus monodisperse polystyrene standards using a GPC column TSKGEL Super HZM-H (Tosoh Corp.) under analysis conditions, flow rate: 0.6 mL/min, eluent: tetrahydrofuran, and column temperature: 40°C.

The compounds used in synthesis are identified below.

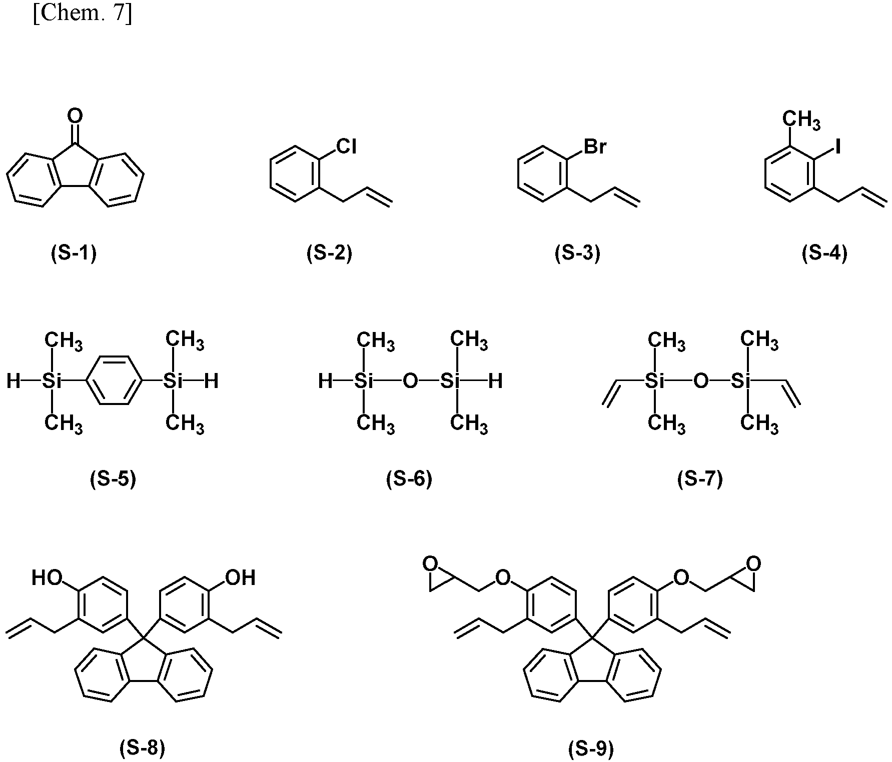

### [Example 1]

A 2-L flask equipped with a thermometer, nitrogen gas inlet tube, reflux condenser, and dropping funnel was charged with 180.2 g (1.00 mol) of the compound having formula (S-1) and 335.8 g (2.20 mol) of the compound having formula (S-2). Then 500 g of chloroform was added to the mixture, which was stirred until the compounds having formulae (S-1) and (S-2) were dissolved. Thereafter, 17.2 g (0.10 mol) of p-toluenesulfonic acid was added to the solution, which was heated at 90°C and stirred for 12 hours. The reaction solution was washed with an aqueous solution of potassium carbonate and deionized water. The solvent was distilled off under reduced pressure, obtaining a solid. By UV/Vis absorption spectroscopy and 1H-NMR spectroscopy (Bruker Corp.), the solid was identified to be a compound having the following formula (4).
¹H-NMR (CDCl₃): δ = 3.27 (4H, d, J=6.3 Hz), 4.76-4.91 (4H, m), 5.78 (2H, ddt, J=17.1, 10.6, 6.3 Hz), 6.53 (2H, d, J=1.2 Hz), 7.22-7.45 (6H, m), 7.52 (2H, d, J=8.3 Hz), 7.64 (2H, dd, J=8.1, 1.4 Hz), 7.85 (2H, dd, J=8.5, 1.5 Hz).

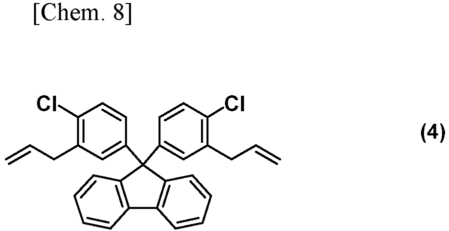

### [Example 2]

A 2-L flask equipped with a thermometer, nitrogen gas inlet tube, reflux condenser, and dropping funnel was charged with 180.2 g (1.00 mol) of the compound having formula (S-1) and 512.4 g (2.60 mol) of the compound having formula (S-3). Then 500 g of chloroform was added to the mixture, which was stirred until the compounds having formulae (S-1) and (S-3) were dissolved. Thereafter, 12.0 g (0.20 mol) of acetic acid was added to the solution, which was heated at 120°C and stirred for 10 hours. The reaction solution was washed with an aqueous solution of sodium hydroxide and deionized water. The solvent was distilled off under reduced pressure, obtaining a solid. By UV/Vis absorption spectroscopy and ¹H-NMR spectroscopy (Bruker Corp.), the solid was identified to be a compound having the following formula (5).
¹H-NMR (CDCl₃): δ = 3.22 (4H, d, J=6.3 Hz), 4.80-4.95 (4H, m), 5.73 (2H, ddt, J=17.1, 10.6, 6.3 Hz), 6.51 (2H, d, J=1.3 Hz), 7.09 (2H, dd, J=8.4, 1.3 Hz), 7.31-7.45 (6H, m), 7.64 (2H, dd, J=8.1, 1.4 Hz), 7.85 (2H, dd, J=8.5, 1.5 Hz).

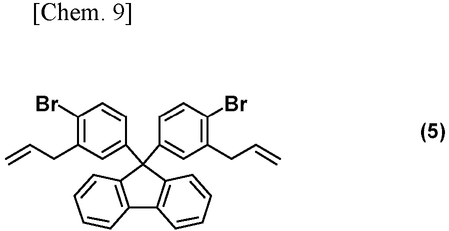

### [Example 3]

A 2-L flask equipped with a thermometer, nitrogen gas inlet tube, reflux condenser, and dropping funnel was charged with 180.2 g (1.00 mol) of the compound having formula (S-1) and 516.2 g (2.00 mol) of the compound having formula (S-4). Then 500 g of chloroform was added to the mixture, which was stirred until the compounds having formulae (S-1) and (S-4) were dissolved. Thereafter, 8.61 g (0.05 mol) of p-toluenesulfonic acid was added to the solution, which was heated at 130°C and stirred for 9 hours. The reaction solution was washed with an aqueous solution of sodium carbonate and deionized water. The solvent was distilled off under reduced pressure, obtaining a solid. By UV/Vis absorption spectroscopy and 1H-NMR spectroscopy (Bruker Corp.), the solid was identified to be a compound having the following formula (6).
¹H-NMR (CDCl₃): δ = 2.35 (6H, s), 3.27 (4H, d, J=6.3 Hz), 4.75-4.90 (4H, m), 5.82 (2H, ddt, J=16.5, 10.6, 6.3 Hz), 6.79 (2H, d, J=1.2 Hz), 6.99 (2H, d, J=1.2 Hz), 7.27-7.45 (4H, m), 7.64 (2H, dd, J=8.1, 1.4 Hz), 7.85 (2H, dd, J=8.5, 1.5 Hz).

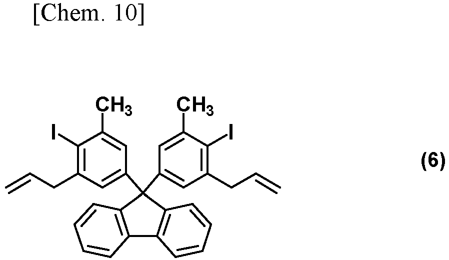

### [Example 4]

A 5-L flask equipped with a stirrer, thermometer, nitrogen purge line, and reflux condenser was charged with 280 g (0.60 mol) of the compound having formula (4), 74.6 g (0.40 mol) of the compound having formula (S-7), and 700 g of toluene. The solution was heated at 70°C. Thereafter, 1.0 g of chloroplatinic acid in toluene (Pt concentration 0.5 wt%) was added and 192 g (0.99 mol) of the compound having formula (S-5) was added dropwise over one hour (total moles of hydrosilyl group/total moles of alkenyl group = 0.99/1). At the end of addition, the reaction solution was heated at 100°C and aged for 4 hours. From the reaction solution, toluene was distilled off under reduced pressure, obtaining Resin 1. Resin 1 had a Mw of 32,000. By ¹H-NMR spectroscopy (Bruker Corp.), Resin 1 was identified to be a polymer having the following formula (7) wherein a=0.6 and b=0.4.

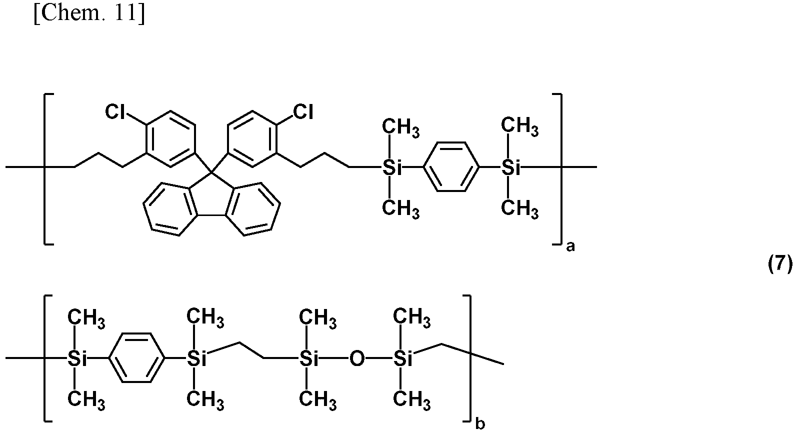

### [Example 5]

A 5-L flask equipped with a stirrer, thermometer, nitrogen purge line, and reflux condenser was charged with 278 g (0.50 mol) of the compound having formula (5), 93.2 g (0.50 mol) of the compound having formula (S-7), and 700 g of toluene. The solution was heated at 70°C. Thereafter, 1.0 g of chloroplatinic acid in toluene (Pt concentration 0.5 wt%) was added and 133 g (0.99 mol) of the compound having formula (S-6) was added dropwise over one hour (total moles of hydrosilyl group/total moles of alkenyl group = 0.99/1). At the end of addition, the reaction solution was heated at 100°C and aged for 6 hours. From the reaction solution, toluene was distilled off under reduced pressure, obtaining Resin 2. Resin 2 had a Mw of 34,000. By ¹H-NMR spectroscopy (Bruker Corp.), Resin 2 was identified to be a polymer having the following formula (8) wherein a=0.5 and b=0.5.

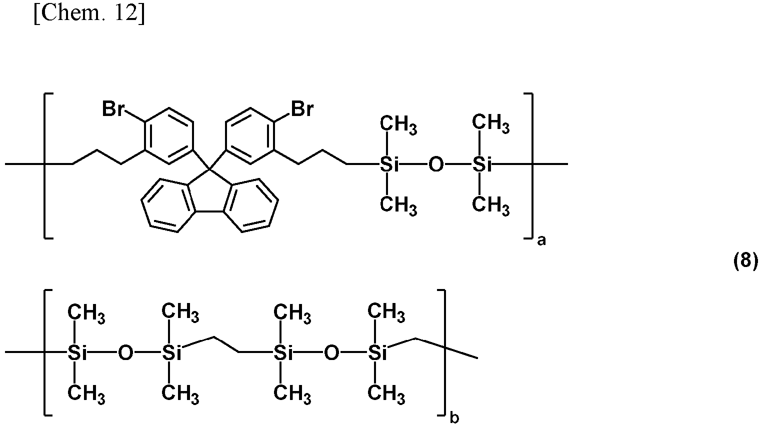

### [Comparative Example 1]

A 5-L flask equipped with a stirrer, thermometer, nitrogen purge line, and reflux condenser was charged with 215 g (0.50 mol) of the compound having formula (S-8), 93.2 g (0.50 mol) of the compound having formula (S-7), and 700 g of toluene. The solution was heated at 70°C. Thereafter, 1.0 g of chloroplatinic acid in toluene (Pt concentration 0.5 wt%) was added and 133 g (0.99 mol) of the compound having formula (S-6) was added dropwise over one hour (total moles of hydrosilyl group/total moles of alkenyl group = 0.99/1). At the end of addition, the reaction solution was heated at 100°C and aged for 14 hours. From the reaction solution, toluene was distilled off under reduced pressure, obtaining Comparative Resin 1. Comparative Resin 1 had a Mw of 30,000. By ¹H-NMR spectroscopy (Bruker Corp.), Comparative Resin 1 was identified to be a polymer having the following formula (9) wherein a=0.5 and b=0.5.

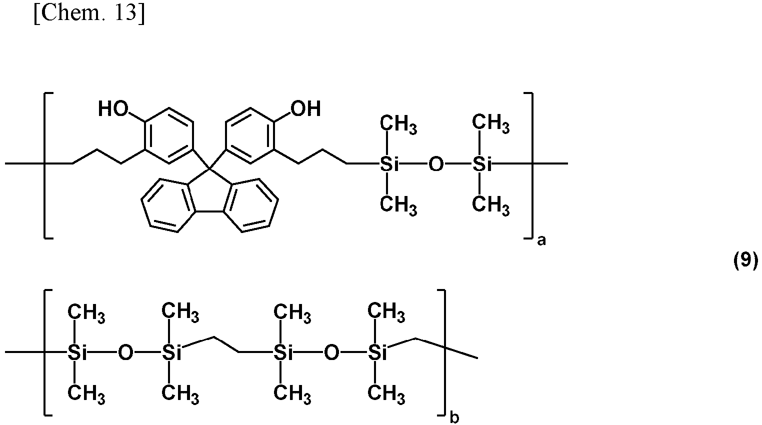

### [Comparative Example 2]

A 5-L flask equipped with a stirrer, thermometer, nitrogen purge line, and reflux condenser was charged with 217 g (0.40 mol) of the compound having formula (S-9), 112 g (0.60 mol) of the compound having formula (S-7), and 700 g of toluene. The solution was heated at 70°C. Thereafter, 1.0 g of chloroplatinic acid in toluene (Pt concentration 0.5 wt%) was added and 192 g (0.99 mol) of the compound having formula (S-5) was added dropwise over one hour (total moles of hydrosilyl group/total moles of alkenyl group = 0.99/1). At the end of addition, the reaction solution was heated at 100°C and aged for 16 hours. From the reaction solution, toluene was distilled off under reduced pressure, obtaining Comparative Resin 2. Comparative Resin 2 had a Mw of 31,000. By ¹H-NMR spectroscopy (Bruker Corp.), Comparative Resin 2 was identified to be a polymer having the following formula (10) wherein a=0.4 and b=0.6.

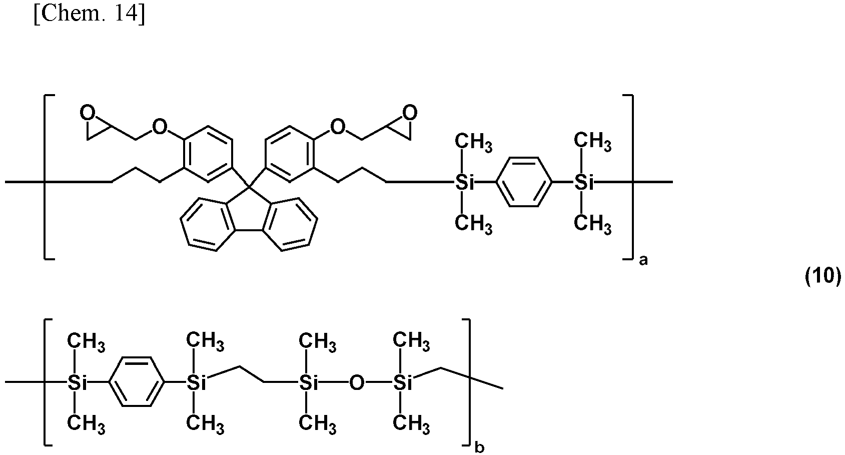

### [Comparison of aging time]

During the synthesis of Resins 1 and 2 and Comparative Resins 1 and 2, the Mw of a product was measured in the course of aging at intervals of 2 hours until Mw exceeded 30,000. The results are shown in Table 1.

**[Table 1]**

| Resin | Mw at intervals of 2 hours in aging | | | | | | | |
|---|---|---|---|---|---|---|---|---|
| | 2 hr | 4 hr | 6 hr | 8 hr | 10 hr | 12 hr | 14 hr | 16 hr |
| Resin 1 | 25000 | 32000 | - | - | - | - | - | - |
| Resin 2 | 24000 | 29000 | 34000 | - | - | - | - | - |
| Comparative Resin 1 | 20000 | 23000 | 25000 | 27000 | 28000 | 29000 | 30000 | - |
| Comparative Resin 2 | 17000 | 20000 | 23000 | 25000 | 26000 | 28000 | 29000 | 31000 |

It is seen from the above results that the fluorene compounds having halogen atoms and allyl groups are synthesized according to the invention. Since the halogen atoms can be readily converted to other substituent groups, the fluorene compounds are useful as a precursor to various fluorene-containing functional materials. The fluorene compounds having halogen atoms and allyl groups exhibit so high reactivity in hydrosilation that the reaction time for polymer synthesis may be shortened.

## Claims

1. A fluorene compound having halogen atoms and allyl groups, represented by the formula (1): 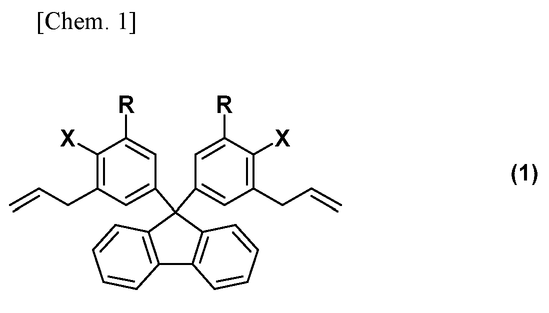 wherein R is each independently hydrogen or methyl and X is each independently fluorine, chlorine, bromine or iodine.

2. The fluorene compound of claim 1 wherein both R are hydrogen.

3. A method for preparing a fluorene compound represented by the formula (1), comprising the step of reacting fluorenone represented by the formula (2) with a compound represented by the formula (3), 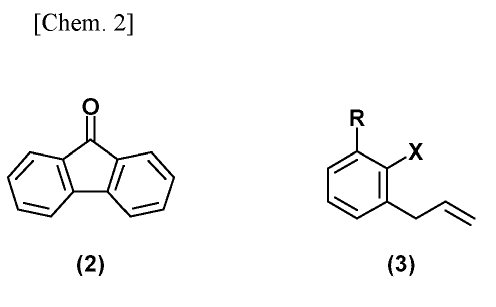 wherein R is hydrogen or methyl and X is fluorine, chlorine, bromine or iodine, 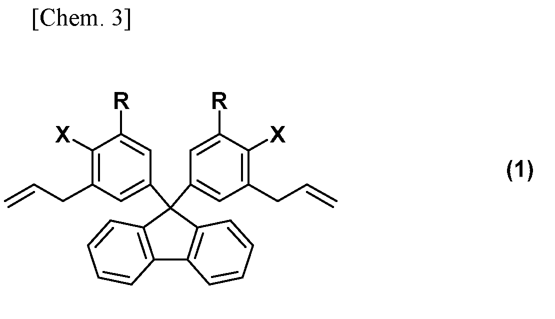 wherein R and X are as defined above.

4. A method for preparing a fluorene skeleton-containing organosilicon compound comprising the step of effecting hydrosilation reaction of the fluorene compound of claim 1 or 2 with an organosilicon compound containing a Si-H group in the presence of a catalyst.
